# EUROPEAN PATENT APPLICATION

(11) **EP 4 389 077 A1**
(43) Date of publication of application: **26.06.2024**
(21) Application number: 23217988.7
(22) Date of filing: 19.12.2023
(51) Int. Cl.: A61F 2/30, A61F 2/38

(54) **ANTI-LUXATION DEVICES FOR A CONSTRAINED PROSTHETIC KNEE**

(30) Priority: 22.12.2022 US 202263434574 P; 08.03.2023 US 202363450878 P
(71) Applicant: Zimmer, Inc., Warsaw, IN 46580 (US)
(72) Inventor: YOKO, Tim, Granger, 46530 (US); VANDIEPENBOS, Jeffery A., New Paris, 46553 (US); BYRD, Brian D., North Webster, 46555 (US); BARKER, Joshua, Warsaw, 46582 (US); BLAYLOCK, Jeff, Fort Wayne, 46807 (US); REIDY, John, Warsaw, 46580 (US)
(74) Representative: Taor, Simon Edward William

(57) **Abstract**

The techniques described herein relate to a prosthesis assembly. The prosthesis assembly includes a femoral component, a tibial bearing component engaged by the femoral component and a tibial baseplate. The tibial baseplate includes a bushing received in a recess in the tibial baseplate and a hinge post coupled to the femoral component and the bushing. The hinge post is at least partially received by the bushing. A prosthesis assembly further includes a capture element coupled to the tibial baseplate. The capture element is engaged by at least one of the bushing or the hinge post to limit distraction of the femoral component from the tibial bearing component and tibial baseplate.

## Description

### CLAIM OF PRIORITY

This application claims the benefit of U.S. Provisional Patent Application Serial No. 63/434,574, filed on December 22, 2022, and also claims the benefit of U.S. Provisional Patent Application Serial No. 63/450,878, filed on March 8, 2023, the benefit of priority of each of which is claimed hereby, and each of which is incorporated by reference herein in its entirety.

### FIELD

The present subject matter relates to orthopedic prostheses and, more particularly, to orthopedic prosthesis used in constrained knee arthroplasties.

### BACKGROUND

Orthopedic procedures and prostheses are commonly utilized to repair and/or replace damaged bone and tissue in the human body. Generally, the knee is formed by the pair of condyles at the distal portion of the femur, the lower surfaces of which bear upon the correspondingly shaped proximal surface plateau of the tibia. The femur and tibia are connected by means of ligaments such as, the posterior cruciate ligament, the lateral collateral ligament, the medial collateral ligament, and the anterior cruciate ligament. These ligaments provide stability to the knee joint.

Prosthetic knee joints can be considered either constrained or unconstrained. For the purposes of this discussion, constrained prosthetic knee systems include femoral and tibial prostheses, which are mechanically linked or constrained to each other to limit relative movement between the femoral and tibial prostheses. Common mechanisms for such mechanical linkage is by a hinge, band or other linkage structure. An unconstrained prosthetic knee system includes femoral and tibial prostheses which are not mechanically linked. An unconstrained knee utilizes the patient's existing ligaments and other soft tissue to provide joint stability. With this in mind, constrained prosthetic knees have particular applicability to cases in which a patient has experienced ligament loss and/or the existing ligaments do not provide adequate support and stability to the knee.

Various constrained knee designs are known. One such design includes a hinge post. This hinge post configuration is positioned within a tibial baseplate (with an end protruding therefrom) and is connected to the femoral component. One hinge post configuration is the NexGen^{®} Rotating Hinge Knee owned by the applicant, for example.

### OVERVIEW

This disclosure pertains generally to improved constrained knee prostheses, particularly those utilizing a hinge post. Some constrained knee prostheses with hinge posts utilize a design where the femoral component (and hinge post) are free to move generally proximal/distal relative to the tibial baseplate and the tibial bearing component. Such arrangement can allow for distraction of the knee j oint. However, the present inventors have recognized that a certain segment of patients receiving a constrained knee prosthesis with the hinge post may have insufficient soft tissue in the knee joint to prevent distraction and then luxation of the femoral component from the tibial baseplate and the tibial bearing component. Luxation can result in pain and other complications for the patient.

Thus, the present inventors have recognized that for the certain segment of the patients with insufficient soft tissue, distraction of the femoral component from the tibial baseplate and the tibial bearing component should be limited. The present inventors recognize various techniques and apparatuses such as a capture element that can interact with the hinge post and/or a bushing of the constrained knee prostheses to limit distraction. As used herein the terms "limiting distraction", "limit distraction", "limits distraction", "limited distraction" or the like includes various prosthesis configurations as further discussed herein. For example, with one example of limited distraction, the femoral component is capable of a certain degree of proximal/distal movement (e.g., movement of a few millimeters to a few centimeters) relative to the tibial baseplate and the tibial bearing component. However, such degree of movement is eventually restricted/halted/stopped such that the femoral component is not capable of further proximal/distal movement such as proximal/distal movement that could result in luxation of the femoral component from the tibial baseplate and the tibial bearing component. This configuration for the prosthesis should be contrasted with configurations of the prosthesis that are capable of "full distraction" where proximal/distal movement of the femoral component relative to the tibial baseplate and the tibial bearing component is not eventually restricted/halted/stopped by a component of the prosthesis and luxation of the knee joint can result if insufficient soft tissue is present. The terms "limiting distraction", "limit distraction", "limits distraction", "limited distraction" or the like includes prosthesis configurations where the proximal/distal movement of the femoral component is substantially entirely limited save for micromotion. The term "micromotion" refers to the small motions that may exist between prosthesis components, such as between the tibial baseplate and capture element, respectively, upon application of force. Such small motions may occur as a result of material deformation in one or both of the interacting components, or may result from slight spaces or clearances therebetween, for example. Micromotion is distinguished from larger movements of components such as the proximal/distal movement of the femoral component relative to the tibial baseplate and the tibial bearing component.

Additional features and benefits of the various examples provided herein will be discussed and/or will be apparent to one of ordinary skill in the art.

To further illustrate the apparatuses, systems and methods disclosed herein, the following non-limiting examples are provided, and which are referred to below as techniques. Parts or all of these examples/techniques can be combined in any manner.

In some aspects, the techniques described herein relate to a prosthesis assembly for a constrained knee including: a femoral component; a tibial bearing component engaged by the femoral component; a tibial baseplate having a distal surface, a proximal surface opposite the distal surface and facing the tibial bearing component, a periphery extending between the proximal surface and the distal surface and a keel extending distally from the distal surface; a bushing received in a recess in the tibial baseplate; a hinge post coupled to the femoral component and the bushing, wherein the hinge post is at least partially received by the bushing; and a capture element coupled to the tibial baseplate, wherein the capture element is engaged by at least one of the bushing or the hinge post to limit distraction of the femoral component from the tibial bearing component and tibial baseplate.

In some aspects, the techniques described herein relate to a prosthesis assembly, wherein the capture element includes one of a snap fit component or a threaded nut configured to be at least partially received in the recess in the tibial baseplate, wherein the capture element has a thru hole configured to allow at least a portion of the hinge post to pass through the capture element.

In some aspects, the techniques described herein relate to a prosthesis assembly, wherein the capture element is spaced a distance proximal of the bushing, and wherein the distance allows for a limited degree of distraction of the femoral component from the tibial bearing component and tibial baseplate.

In some aspects, the techniques described herein relate to a prosthesis assembly, wherein the capture element is proximal to and abuts a proximal end of the bushing and thereby entirely limits distraction of the femoral component from the tibial bearing component and tibial baseplate.

In some aspects, the techniques described herein relate to a prosthesis assembly, wherein hinge post is coupled to the bushing by first threads that couple with second threads of the bushing.

In some aspects, the techniques described herein relate to a prosthesis assembly, wherein coupling of the first threads with the second threads positions the bushing a desired location with respect to the capture element.

In some aspects, the techniques described herein relate to a prosthesis system for a constrained knee including: a femoral component; a tibial bearing component configured to articulate with the femoral component; a tibial baseplate having a distal surface, a proximal surface opposite the distal surface and facing the tibial bearing component, a periphery extending between the proximal surface and the distal surface and a keel extending distally from the distal surface; a bushing configured to insert into a recess in the tibial baseplate; a hinge post configured to couple with the femoral component and configured to couple with the bushing; and a capture element configured to couple with the tibial baseplate and having a thru hole configured to allow at least a portion of the hinge post to pass therethrough, wherein, when coupled to the tibial baseplate, the capture element is configured to be engaged by at least one of the bushing or the hinge post to limit distraction of the femoral component from the tibial bearing component and tibial baseplate.

In some aspects, the techniques described herein relate to a prosthesis system, wherein hinge post is configured to be coupled with the bushing by first threads that engage second threads of the bushing.

In some aspects, the techniques described herein relate to a prosthesis system, wherein, when the first threads are coupled with the second threads positions the bushing is positioned at a desired location relative to the capture element.

In some aspects, the techniques described herein relate to a prosthesis system, wherein the capture element is configured to abut or is spaced a distance from the bushing when the bushing is positioned in the desired location.

In some aspects, the techniques described herein relate to a prosthesis system, wherein the capture element includes one of a snap fit component or a threaded nut configured to be at least partially received in the recess in the tibial baseplate.

In some aspects, the techniques described herein relate to a prosthesis system, wherein, when assembled with the bushing, the capture element is spaced a distance proximal of the bushing, and wherein the distance allows for a limited degree of distraction of the femoral component from the tibial bearing component and tibial baseplate.

In some aspects, the techniques described herein relate to a prosthesis system, wherein, when assembled with the bushing, the capture element is proximal to and abuts a proximal end of the bushing and thereby entirely limits distraction of the femoral component from the tibial bearing component and tibial baseplate.

In some aspects, the techniques described herein relate to a method of assembling a prosthesis assembly for a constrained knee, the method including: coupling a hinge post with a femoral component; inserting a bushing into a recess in a tibial baseplate; positioning a capture element within the recess of the tibial baseplate; inserting the hinge post when coupled to the femoral component into the recess and through the capture element; coupling the hinge post with the bushing within the recess; and inserting a tibial bearing component between the femoral component and the tibial baseplate.

In some aspects, the techniques described herein relate to a method, wherein positioning the capture element within the recess of the tibial baseplate includes deflecting the capture element to engage one or more retention features of the tibial baseplate.

In some aspects, the techniques described herein relate to a method, wherein positioning the capture element within the recess of the tibial baseplate includes inserting the capture element into a receptacle that is adjacent to and communicates with the recess and moving the capture element laterally from the receptacle to the recess.

In some aspects, the techniques described herein relate to a method, wherein positioning the capture element within the recess of the tibial baseplate includes threading the capture element into the tibial baseplate.

In some aspects, the techniques described herein relate to a method, wherein coupling the hinge post with the bushing within the recess positions the bushing at a desired location relative to the capture element.

In some aspects, the techniques described herein relate to a method, further including engaging the capture element with at least one of the bushing or the hinge post to limit distraction of the femoral component from the tibial bearing component and tibial baseplate.

In some aspects, the techniques described herein relate to a prosthesis assembly, wherein the capture element is initially coupled to the bushing by a frangible connection, wherein the frangible connection is severed by threading of the hinge post with the bushing to separate the capture element from the bushing.

In some aspects, the techniques described herein relate to a prosthesis system, wherein the capture element is initially coupled to the bushing by a frangible connection, wherein the frangible connection is severed by threading of the hinge post with the bushing to separate the capture element from the bushing.

In some aspects, the techniques described herein relate to a method, wherein coupling the hinge post with the bushing within the recess severs a connection between the capture element and the bushing.

### BRIEF DESCRIPTION OF THE DRAWINGS

In the drawings, which are not necessarily drawn to scale, like numerals can describe similar components in different views. Like numerals having different letter suffixes can represent different instances of similar components. The drawings illustrate generally, by way of example, but not by way of limitation, various examples discussed in the present document.
FIGS. 1 and 2 illustrate knee joint structures providing suitable environments in which the constrained prosthesis assemblies in accordance with an example of the present application can be utilized.
FIG. 3 is a perspective view of a constrained knee prosthesis assembly according to an example of the present application.
FIG. 3A is a perspective view of the constrained knee prosthesis assembly of FIG. 3 undergoing distraction with a femoral component moving proximal/distal movement relative to a tibial baseplate and a tibial bearing component according to an example of the present application.
FIG. 4 is an exploded view of the constrained knee prosthesis assembly of FIG. 3.
FIG. 5 is a cross-sectional view of a second example of a constrained knee prosthesis assembly with a capture element configured to limit distraction of the femoral component relative to the tibial baseplate and/or the tibia bearing component according to an example of the present application.
FIG. 6 is a top plan view of the tibial baseplate and the capture element of FIG. 5 being moved into a desired position within a recess within the tibial baseplate to limit distraction according to an example of the present application.
FIG. 7 shows various perspective views of examples of the capture element according to an example of the present application.
FIG. 8 is a cross-sectional view along a sagittal plane of the tibial baseplate and the tibial bearing component of the constrained knee prosthesis assembly similar to that of FIG. 5 but using another example of a capture element that is snap-fit within at least a portion of a recess of the tibial baseplate according to an example of the present application.
FIG. 9A is a cross-sectional view of the tibial baseplate and the tibial bearing component of a third constrained knee prosthesis assembly with yet another example of a capture element coupled to the tibial baseplate with threads according to an example of the present application.
FIG. 9B shows a perspective view of the capture element of FIG. 9A in isolation having the threads along an outer diameter and a thru hole and other features according to an example of the present application.
FIG. 10 is a cross-sectional view of the tibial baseplate and the tibial bearing component of a fourth constrained knee prosthesis assembly with a hinge post and a bushing having a modified configuration as compared with previous examples.
FIG. 11 is a cross-sectional view of the tibial baseplate and the tibial bearing component of a fifth constrained knee prosthesis assembly with at least the bushing having a modified configuration as compared with the example of FIG. 10 according to an example of the present application.
FIG. 11A is a perspective view of proximal portions of the capture element and the bushing of FIG. 11.
FIG. 11B is a perspective view of the capture element and the bushing of FIG. 11 in isolation and spaced relative to one another to allow for a limited distraction according to an example of the present application.
FIG. 12 is a cross-sectional view of the tibial baseplate and the tibial bearing component of a sixth constrained knee prosthesis assembly with at least the hinge post having a modified configuration to include an engagement feature as compared with previous examples.
FIG. 13 is a perspective view of another example of a hinge post that can be used with the constrained knee prosthesis assemblies discussed herein including those of FIGS. 14-19.
FIG. 14 is a perspective view of another example of a capture element and a bushing in isolation and spaced relative to one another to allow for a limited distraction according to an example of the present application.
FIG. 15 illustrates a process of switching a constrained knee prosthesis from a bushing that allows for full distraction to the bushing and capture element of FIG. 14 that provides from limited distraction according to an example of the present application.
FIG. 16 is a perspective view of yet another example of a capture element and a bushing in isolation and spaced relative to one another to allow for a limited distraction according to an example of the present application.
FIG. 17 shows a cross-sectional view of the tibial baseplate and the tibial bearing component of a seventh constrained knee prosthesis assembly with the hinge post of FIG. 13 and the examples of the hinge post and the capture element of FIG. 16.
FIG. 18 is a perspective view of yet another example of a capture element and a bushing in isolation and spaced relative to one another to allow for a limited distraction according to an example of the present application.
FIG. 19 illustrates a process of switching components of the seventh constrained knee prosthesis of the from a bushing that allows for full distraction to the bushing and capture element of FIG. 18 that provides from limited distraction according to an example of the present application.
FIG. 20 is a plan view of a hinge post that can be used with the constrained prosthesis assemblies discussed herein including those of FIGS. 21A-23.
FIG. 21A is a plan view of another example of a capture element that combines a proximal capture element and a bushing initially assembled together according to an example of the present application.
FIG. 21B is a perspective view of the capture element of FIG. 21A.
FIG. 21C is a cross-sectional view of the capture element of FIGS. 21A and 21B.
FIG. 22 is a perspective view of the capture element and the bushing of FIGS. 21A-21C now separated from one another and shown spaced relative to one another to allow for a limited distraction according to an example of the present application.
FIG. 23 shows a cross-sectional view of the tibial baseplate and the tibial bearing component of a eighth constrained knee prosthesis assembly with the hinge post of FIG. 20 and the examples of the hinge post and the capture element of FIG. 22 configured to limit distraction of a femoral component from the tibial baseplate and the tibial bearing component.

### DETAILED DESCRIPTION

The present application relates to constrained tibial prosthesis assemblies and systems including tibial baseplates, capture elements, bushings and hinge posts among other components. This application focuses on limiting distraction of the femoral component relative to the tibial baseplate and/or the tibial bearing component through various features, techniques and component including the capture elements discussed herein. Limiting distraction can prevent luxation of the knee joint and corresponding pain, discomfort and a possible need for medical intervention.

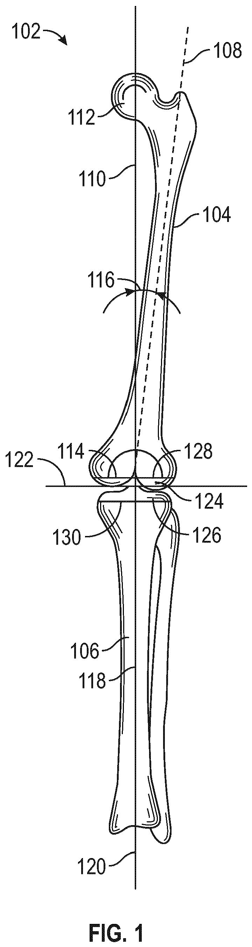

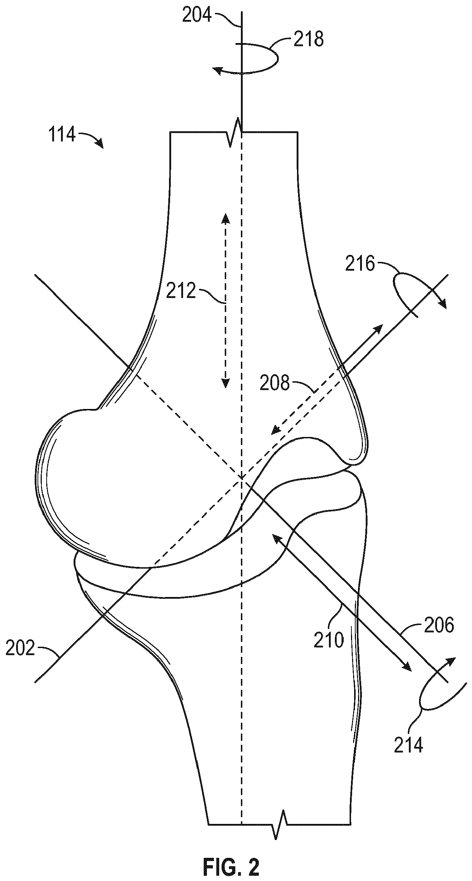

To better understand knee joint replacement procedures, it can be helpful to understand the relationship of bones and bone cuts that can be made to orient various provisional and permanent prosthesis components within a knee joint. FIGS. 1 and 2 illustrate several features of knee joint structures and orientations. In FIG. 1, a frontal view of a lower limb 102, including a femur 104 and a tibia 106, is shown to illustrate various lower limb axes. The femur 104 has an anatomic axis 108 that coincides generally with its intramedullary canal. The femur 104 also has a mechanical axis 110, or load axis, running from the center of a femoral head 112 to the center of a knee joint 114. The angle 116 extending between these two axes varies among the patient population, but is generally on the order of between 5-7 degrees, inclusive. Like the femur 104, the tibia 106 also has an anatomic axis coinciding generally with its intramedullary canal. The mechanical axis 118 of the tibia 106 runs from the center of the knee joint 114 to the center of an ankle region 120 and is generally collinear with its anatomic axis.

A joint line 122, about which the knee joint 114 flexes, is approximately parallel to a line through medial and lateral femoral condyles 124 and to a tibial plateau 126. Although illustrated as perpendicular in FIG. 1, the joint line 122 can extend at a varus or valgus angle relative to the mechanical axes 110 and 118 of the femur 104 and tibia 106, respectively. Normally, during a partial or total knee replacement procedure, portions of a distal end of the femur 104 or a proximal end of the tibia 106 are resected to be parallel or approximately parallel to the joint line 122, and thus perpendicular to the mechanical axes 110 and 118, as indicated at 128 and 130, respectively.

FIG. 2 illustrates a closer view of the knee joint 114 and its coordinate system, in which a medial/lateral axis 202 corresponds approximately to the joint line 122 (FIG. 1), a proximal/distal axis 204 corresponds approximately to the mechanical axes 110 and 118 (FIG. 1), and an anterior/posterior axis 206 is approximately normal to the other two axes. Position along each of these axes can be depicted by arrows, which can represent the medial/lateral 208, anterior/posterior 210, and proximal/distal 212 positioning of inserted prosthesis components. Rotation about each of these axes can also be depicted by arrows. Rotation about the proximal/distal axis 204 can correspond anatomically to external rotation of a femoral component, while rotation about the anterior/posterior axis 206 and medial/lateral axis 202 can correspond to extension plane slope and varus/valgus angle of a component, respectively. Depending on a position of the proximal tibial cut 130 (FIG. 1) made, a varus/valgus angle 214, extension plane angle 216, external rotation 218, or joint extension gap can be affected. Similarly, a position of the distal femoral cut 128 (FIG. 1) can affect the location of the joint line 122, the extension gap, the varus/valgus angle 214, or the extension plane angle 216.

As used herein, the terms "proximal" and "distal" should be given their generally understood anatomical interpretation. The term "proximal" refers to a direction generally toward the torso of a patient, and "distal" refers to the opposite direction of proximal, i.e., away from the torso of a patient. It should be understood that the use of the terms "proximal" and "distal" should be interpreted as though the patient were standing with the knee joint in extension. The intent is to differentiate the terms "proximal" and "distal" from the terms "anterior" and "posterior". As used herein, the terms "anterior" and "posterior" should be given their generally understood anatomical interpretation. Thus, "posterior" refers to a rear of the patient, e.g., a back of the knee. Similarly, "anterior" refers to a front of the patient, e.g., a front of the knee. Thus, "posterior" refers to the opposite direction of "anterior". Similarly, the term "lateral" refers to the opposite direction of "medial". The term "medial/lateral" means medial to lateral or lateral to medial. The term "proximal/distal" means proximal to distal or distal to proximal. The term "anterior/posterior" means anterior to posterior or posterior to anterior.

As used herein, the "periphery" of a tibial baseplate refers to any periphery as viewed in a top plan view, e.g., in a generally transverse anatomical plane. Alternatively, the periphery of a tibial baseplate may be any periphery as viewed in bottom plan view, e.g., in a generally transverse plane and looking at the distal surface adapted to contact a resected proximal surface of a tibial bone.

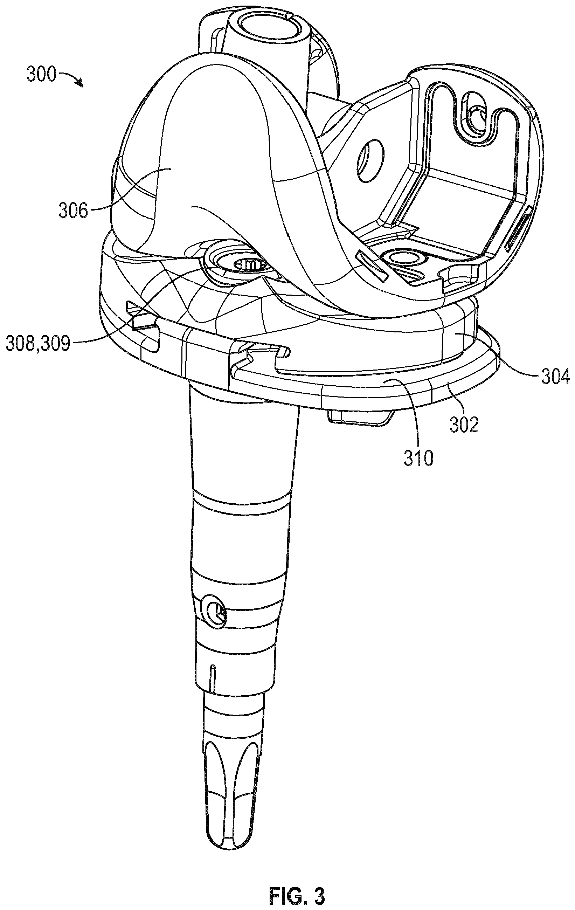

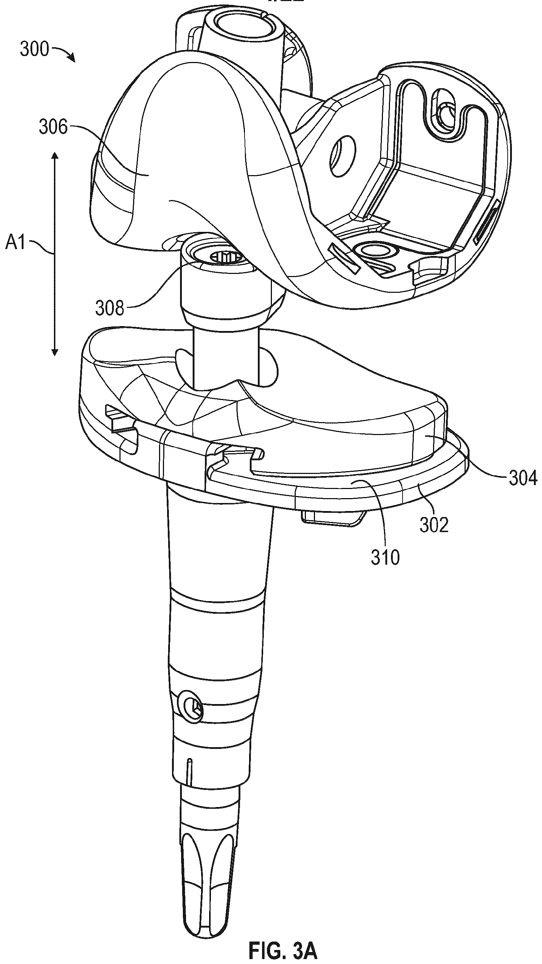

FIG. 3 shows a prosthesis assembly 300 for a constrained knee. The prosthesis assembly 300 can include a tibial baseplate 302, a tibial bearing component 304 (sometime called a meniscal component, poly, articular component or bearing), a femoral component 306 and a hinge post 308.

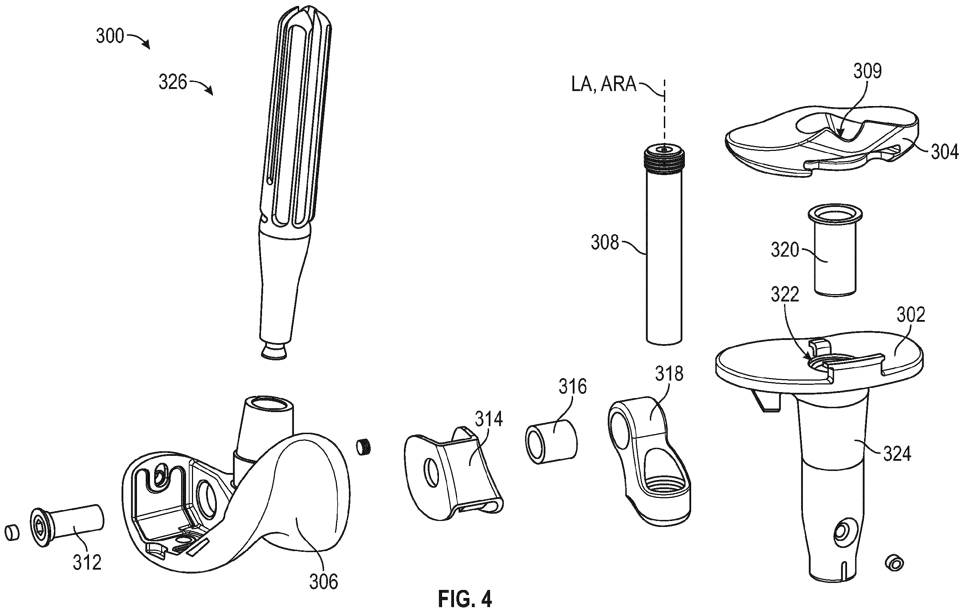

The tibial bearing component 304 can be coupled to and can be positioned atop a proximal surface 310 of the tibial baseplate 302. The tibial bearing component 304 can be formed of polymer material such as Ultra-High-Molecular-Weight-Polyethylene ("UHMWPE"), etc. The tibial bearing component 304 can be configured to articulate with the femoral component 306 through knee joint flexion and extension as known in the art. The prosthesis assembly 300 has the femoral component 306 and the tibial baseplate 302 mechanically linked to one another. This is accomplished by the hinge post 308 and other components further illustrated and discussed in FIG. 4. The hinge post 308 is coupled to the femoral component 306 and is received within a recess 309 of the tibial bearing component 304 and a recess 322 (FIG. 4) of the tibial baseplate 302.

FIG. 3A shows distraction of the knee joint. In particular, the femoral component 306 is moved generally proximal/distal (as indicated by arrow A1) from the tibial bearing component 304 and the tibial baseplate 302. Luxation of the knee joint can result if insufficient soft tissue remains in the knee joint to retain the femoral component 306 on the tibial bearing component 304 in the manner shown in FIG. 3. Movement between the femoral component 306 and the tibial bearing component 304 and the tibial baseplate 302 creates a distance (gap) between the femoral component 306 and an articular surface of the tibial bearing component 304. As the hinge post 308 remains at least partially retained within recesses of the tibial baseplate 302 and/or the tibial bearing component 304 in the example of FIG. 3A, distraction of the femoral component 306 from the tibial bearing component 304 and the tibial baseplate 302 is generally along the proximal/distal axis 204 and along the direction 212 as indicated in FIG. 2. However, should the hinge post 308 come free of the recesses 309 and 322 (FIG. 4), a luxation of the knee joint can result. Luxation can be painful and can result in other complications for the patient.

FIG. 4 shows an exploded view of the prosthesis assembly 300, the tibial baseplate 302, the tibial bearing component 304, the femoral component 306, the hinge post 308 and further illustrates a hinge axle 312, poly box 314, axle bushing 316, shackle 318 and a bushing 320.

The hinge post 308 is connected to femoral component 306 via the shackle 318, the axle bushing 316 and the hinge axle 312. A distal portion of the shackle 318 is received in the recess 309 in the tibial bearing component 304 and the distal portion is threaded or otherwise connected to the hinge post 308. The hinge post 308 extends distally through the recess 309 of the tibial bearing component 304 and is received in a recess 322 of the tibial baseplate 302. The recess 322 of the tibial baseplate 302 that receives the hinge post 308 can at least partially be formed by a keel 324 of the tibial baseplate 302. The hinge post 308 can be moveable (e.g., rotatable and/or capable of distraction as shown in FIG. 3A) relative to the tibial bearing component 304 and/or the tibial baseplate 302. The hinge post 308 can be rotatably connected to femoral component 306 via the hinge axle 312. Thus, a longitudinal axis LA that defines a centerline of the hinge post 308 can define an axis of rotation/articulation ARA for the knee joint as the femoral component 306 and the tibial baseplate 302 are mechanically linked.

When assembled, the shackle 318 can be placed between opposing walls of poly box 314. When assembled on the hinge axle 312, the axle bushing 316 additionally resides within an aperture on a proximal portion of the shackle 318. The shackle 318 and hinge post 308 can be formed from suitable materials such as a titanium alloy, a cobalt-chromium alloy, etc. while the axle bushing 316 and the poly box 314 can be formed from a different materials such as plastic, e.g., UHMWPE. The axle bushing 316 acts as a bearing between the shackle 318 and the hinge axle 312. The poly box 314 acts as a bearing between the femoral component 306 and the shackle 318.

The prosthesis assembly 300 of FIG. 4 shows a system 326 of components where distraction of the knee is not limited by a capture element or other feature of the prosthesis assembly 300. Thus, the system 326 provides components that when assembled are configured for full distraction. Thus, soft tissue of the knee is relied upon to limit distraction between the femoral component 306 and the tibial baseplate 302 and the tibial bearing component 304 as discussed previously. The bushing 320 can be configured to insert into the recess 322 of at least the tibial baseplate 302. The bushing 320 can also insert into or through the recess 309 (FIG. 3) of the tibial bearing component 304 in some examples. The bushing 320 can be configured to receive at least a portion of the hinge post 308. The bushing 320 can act as a bearing between the hinge post 308 and the tibial baseplate 302. The hinge post 308 can be moveable generally proximal/distal relative to the bushing 320 such as during the distraction portrayed in FIG. 3A.

Determining if full joint distraction is desirable based upon the patient anatomy. This determination process can include ascertaining a condition, functionality and amount of soft tissue in the knee joint including the number and condition of any remaining ligaments. The determination process can also include the physician distracting the knee joint, performing a range of motion of the knee joint, measuring gaps between bone and/or soft tissue within the knee joint such as measuring the joint extension gap discussed in reference to FIGS. 1 and 2, or the like. Some or all of these determinations can be performed prior to or after implantation of femoral component, the tibial bearing component, the tibial baseplate and/or other components.

If the physician determines that, based upon the patient anatomy, the knee joint is sufficient to maintain the femoral component mechanically linked to the tibial baseplate (e.g., a luxation of the knee joint will likely not result) during full distraction, the physician can implement a first constrained prosthesis assembly configured to allow for full distraction of the femoral component from the tibial bearing component and/or the tibial baseplate. Alternatively, if the physician determines that, based upon the patient anatomy, the knee joint is insufficient to maintain the femoral component mechanically linked to the tibial baseplate (e.g., a luxation of the knee joint could possibly result) during full distraction, the physician can implement 408 a second constrained prosthesis assembly configured to allow for limited distraction of the femoral component from the tibial bearing component and/or the tibial baseplate.

According to one example, a method of assembling a prosthesis assembly for a constrained knee is discussed and shown herein. The method can include: coupling a hinge post with a femoral component, inserting a bushing into a recess in a tibial baseplate, positioning a capture element within the recess of the tibial baseplate, inserting the hinge post when coupled to the femoral component into the recess and through the capture element, coupling the hinge post with the bushing within the recess and inserting a tibial bearing component between the femoral component and the tibial baseplate.

The method can optionally include that the positioning the capture element within the recess of the tibial baseplate can include deflecting the capture element to engage one or more retention features of the tibial baseplate. The positioning the capture element within the recess of the tibial baseplate can include inserting the capture element into a receptacle that is adjacent to and communicates with the recess and moving the capture element laterally from the receptacle to the recess. The positioning the capture element within the recess of the tibial baseplate can include threading the capture element into the tibial baseplate. Coupling the hinge post with the bushing within the recess can position the bushing at a desired location relative to the capture element. Engaging the capture element with at least one of the bushing or the hinge post can limit distraction of the femoral component from the tibial bearing component and tibial baseplate.

It should be noted that the step of inserting a tibial bearing component between the femoral component and the tibial baseplate can be performed prior to or after the steps of coupling a hinge post with a femoral component, inserting a bushing into a recess in a tibial baseplate, positioning a capture element within the recess of the tibial baseplate and/or inserting the hinge post when coupled to the femoral component into the recess and through the capture element, coupling the hinge post with the bushing within the recess. Thus, the method described above need not be limited in the order of the steps described.

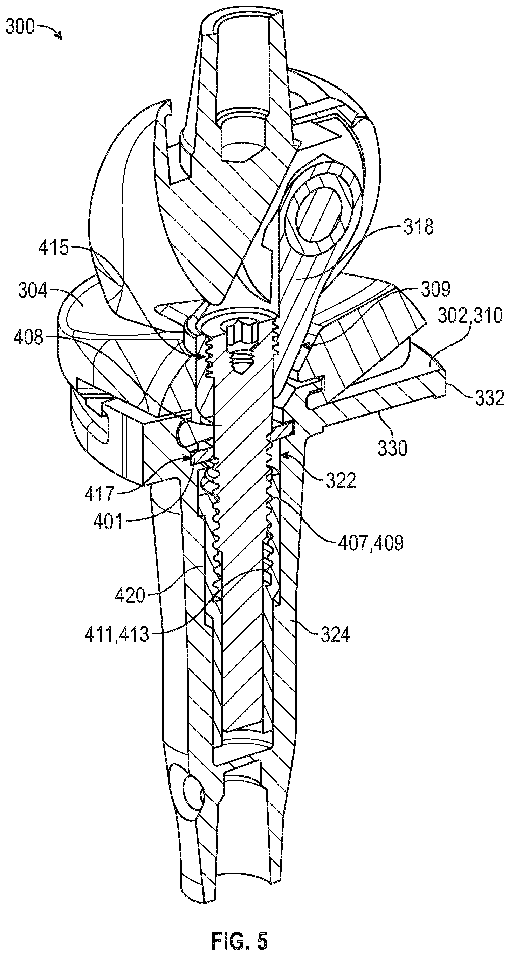

FIG. 5 is a cross-section of a prosthesis assembly 400 having a construction similar to that of the prosthesis assembly 300 described previously. However, the prosthesis assembly 400 differs and includes a capture element 401 that limits distraction of the femoral component 306 from the tibial bearing component 304 and the tibial baseplate 302. At least the tibial baseplate 302 can be configured to receive and/or couple with the capture element 401 as further discussed herein.

The cross-section of FIG. 5 shows a distal surface 330 of the tibial baseplate 302 as well as the proximal surface 310 and a periphery 332. The periphery 332 extends around the tibial baseplate 302 between the proximal surface 310 and the distal surface 330. The keel 324 extends distally from the distal surface 330 of the tibial baseplate 302. The keel 324 at least partially defines the recess 322 that receives a hinge post 408 and a bushing 420. The keel 324 can be integral with (monolithic) or can be attached to (e.g. via thread or another mechanical connection mechanism) a remainder of the tibial baseplate 302. The keel 324 can be configured to extend distally and can be shaped to fit in an intermedullary canal of the tibia 106 (FIG. 1) to provide fixation for the tibial baseplate 302.

The distal surface 330 can include features such as threaded apertures for the connection of pegs, augments or other components as known in the art. Distal surface 330 (and other features of the tibial baseplate such as the keel 324) can be made of a porous or highly porous material that facilitates an amount of bone ingrowth. A highly porous biomaterial is useful as a bone substitute and as cell and tissue receptive material. A highly porous biomaterial may have a porosity as low as 30%, 55%, or as high as 70%, 80%, 85%, or 90%. The highly porous material can have an average pore size of between 100 microns and 1000 microns, for example. However, use of the highly porous biomaterial is not contemplated in all examples. For example, material such as bone cement can be utilized as an alternative to the highly porous biomaterial.

An example of such porous or highly porous material is OsseoTi^{®} generally available from Zimmer Biomet, Inc., of Warsaw, Ind. The material can include titanium or titanium alloy and can additionally include other materials. Such material (including a base of relatively less porous or non-porous biocompatible material) can be manufactured using additive manufacturing processes such as laser sintering or the like. OsseoTi^{®} is highly biocompatible, has high corrosion resistance and includes a highly interconnected porous architecture that mimics the porous structure of human cancellous bone, which can enhance bone integration and in-growth. The porous or highly porous material can be manufactured to be layered over or otherwise structured with/on a relatively less porous or non-porous biocompatible material such as titanium, titanium alloy, stainless steel or other material as known in the art.

Another example of such a porous or highly porous material is produced using Trabecular Metal^{™} Technology generally available from Zimmer Biomet, Inc., of Warsaw, Ind. Such a material may be formed from a reticulated vitreous carbon foam substrate which is infiltrated and coated with a biocompatible metal, such as tantalum, by a chemical vapor deposition ("CVD") process in the manner disclosed in detail in U.S. Pat. No. 5,282,861 to Kaplan, the entire disclosure of which is hereby expressly incorporated herein by reference. In addition to tantalum, other metals such as niobium, or alloys of tantalum and niobium with one another or with other metals may also be used. The porous tantalum structure may be made in a variety of densities in order to selectively tailor the structure for particular applications. In particular, as discussed in the above-incorporated U.S. Pat. No. 5,282,861, the porous tantalum may be fabricated to virtually any desired porosity and pore size, and can thus be matched with the surrounding natural bone in order to provide an improved matrix for bone ingrowth and mineralization.

Generally, the porous material structures contemplated can include a large plurality of ligaments defining open spaces there between, with each ligament generally including a core covered by a thin film of metal. The open spaces between the ligaments form a matrix of continuous channels having no dead ends, such that growth of cancellous bone through the porous tantalum structure is uninhibited. The porous or highly porous material may include up to 70%, 85%, or more void space therein. Thus, porous or highly porous material is a lightweight, strong porous structure which is substantially uniform and consistent in composition, and closely resembles the structure of natural cancellous bone, thereby providing a matrix into which cancellous bone may grow to provide fixation of the tibial baseplate to the patient's bone.

FIG. 5 shows the prosthesis assembly 400 can include a hinge post 408 and a bushing 420 that are modified compared with the hinge post 308 and the bushing 320 of the example of FIG. 4. The bushing 420 can be configured to receive at least a portion of the hinge post 408 therein. The hinge post 408 can include engagement features 407 such as threads 409. These engagement features 407 can be configured to couple with corresponding engagement features 411 such as threads 413 of the bushing 420. The hinge post 408 and the bushing 420 can be coupled for movement together via the engagement features 407, 411. This configuration can differ from the construction of the hinge post 308 and the bushing 320 of the example of FIG. 4, which are not coupled for movement together. Thus, the hinge post 308 and the bushing 320 do not include engagement features such as threads. The hinge post 308 can be moveable relative to the bushing 320.

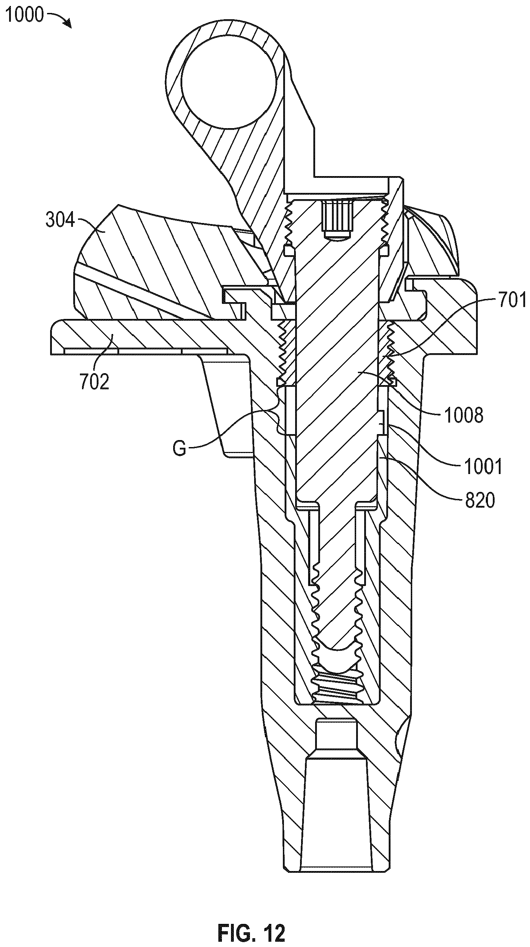

FIG. 5 shows the hinge post 408 coupled with the shackle 318 at a proximal end thereof. This can be via a thread connection 415 between the hinge post 408 and the shackle 318 that allows the hinge post 408 and the shackle 318 to be reversibly coupled to and decoupled from one another allowing for change in the configuration of the hinge post (e.g., a change from the hinge post 308 to the hinge post 408, for example, to facilitate limited distraction). The shackle 318 and the hinge post 408 can extend into and be received by the recess 309 of the tibial bearing component 304. The hinge post 408 can have the engagement features 407 (here threads 409) arranged along a middle or distal portion of the hinge post 408. The threads 409 and threads 413 can be rope threads, knuckle threads, ACME threads or other types of threads as known in the art. In some examples, the threads 409 can differ slightly in pitch or another geometric aspect from the threads 413 so as to create a slight interference. This slight interference can draw the bushing 420 to a desired position within the recess 322 relative to the capture element 401. This position for the bushing 420 relative to the capture element 401 can allow for a desired amount of limited distraction between the femoral component 306 and the tibial bearing component 304 and the tibial baseplate 302 prior to engagement of the bushing 420 with the capture element 401. Although the example of FIG. 5 contemplates that engagement between the bushing 420 and the capture element 401 halts/stops the limited distraction (movement) of the femoral component 306 relative to the tibial bearing component 304 and the tibial baseplate 302, other examples such as those of FIG. 12 herein and also those described in U.S. Provisional Patent Entitled, ANTI-LUXATION DEVICES FOR A CONSTRAINED PROSTHETIC KNEE, filed on the even day herewith, the entire contents of which are incorporated herein by reference, contemplate that engagement features (e.g., barbs, tabs, ribs or other type of projections) are part of the hinge post and these engagement features engage the capture element 401 to halt/stop distraction of the femoral component 306 relative to the tibial bearing component 304 and the tibial baseplate 302. Thus, the capture element 401 is engaged by at least one of the bushing 420 and/or the hinge post 408 to limit distraction of the femoral component 306 from the tibial bearing component 304 and the tibial baseplate 302.

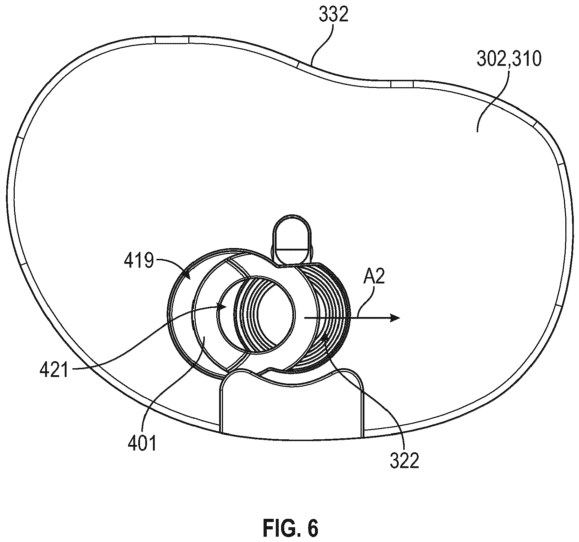

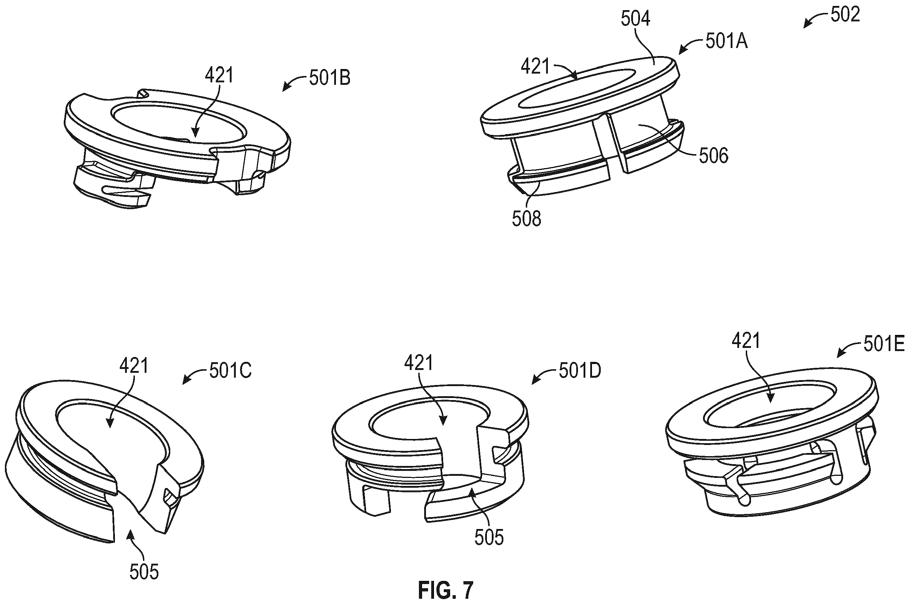

In FIG. 5, the capture element 401 can be positioned at least partially within the recess 322 of the tibial baseplate 302 such as proximal of the bushing 420. The capture element 401 can have a thru hole (see e.g., FIGS. 6 and 7) configured to receive at least a portion of the hinge post 408. Indeed, the capture element 401 with the thru hole can be configured to allow the hinge post 408 to pass therethrough and extend distally to engage and couple with the bushing 420 as shown in FIG. 5. The capture element 401 can comprise a component that, when positioned at least partially within the recess 322 and coupled with the tibial baseplate 302, can halt/restrict movement of the hinge post 408.

FIG. 5 shows the capture element 401 can be ring shaped and can be received in a groove 417 in the tibial baseplate 302. The groove 417 and the capture element 401 can be located distal of the proximal surface 310 of the tibial baseplate 302 such that the capture element 401 is distal of the tibial bearing component 304. The groove 417 can communicate with and can extend outward of the recess 322. At least a portion of the capture element 401 can extend into the recess 322 and can interface with the hinge post 408. This portion of the capture element 401 that is positioned in the recess 322 can be engaged by the bushing 420 during limited distraction as described previously.

FIG. 6 shows a top view of the tibial baseplate 302, and hence, shows the proximal surface 310 and the periphery 332. The proximal surface 310 and periphery 332 can have a particular asymmetry, with respect to a medial/lateral centerline. This shape is designed to maximize tibial coverage for a large proportion of knee-replacement candidates. The asymmetric shape results in a medial compartment of the tibial baseplate being relatively larger than the lateral compartment of the tibial baseplate 302. Maximized coverage of cortical bone facilitates superior support of tibial baseplate 302. A firm, enduring fixation of tibial baseplate 302 to tibia is facilitated by large-area contact between the cortical and cancellous bone of tibia.

FIG. 6 shows the capture element 401 in the process of being assembled with the tibial baseplate 302 (including by being inserted into the recess 322). The hinge post 408, shackle, femoral component and the bushing 420 are not shown in FIG. 6 for clarity. The assembly process includes inserting the capture element 401 into a receptacle 419 that is adjacent to and communicates with the recess 322 and the groove 417 (FIG. 5). The process includes moving (as indicated by arrow A2) the capture element 401 generally medially or laterally from the receptacle 419 to the recess 322 (e.g., fully into the groove 417 to the position of FIG. 5).

FIG. 6 additionally illustrates a thru hole 421 of the capture element 401. The thru hole 421 is configured to allow at least a portion of the hinge post 408 (FIG. 5) to pass therethrough into a remainder of the recess 322 and to the bushing 420 (FIG. 5).

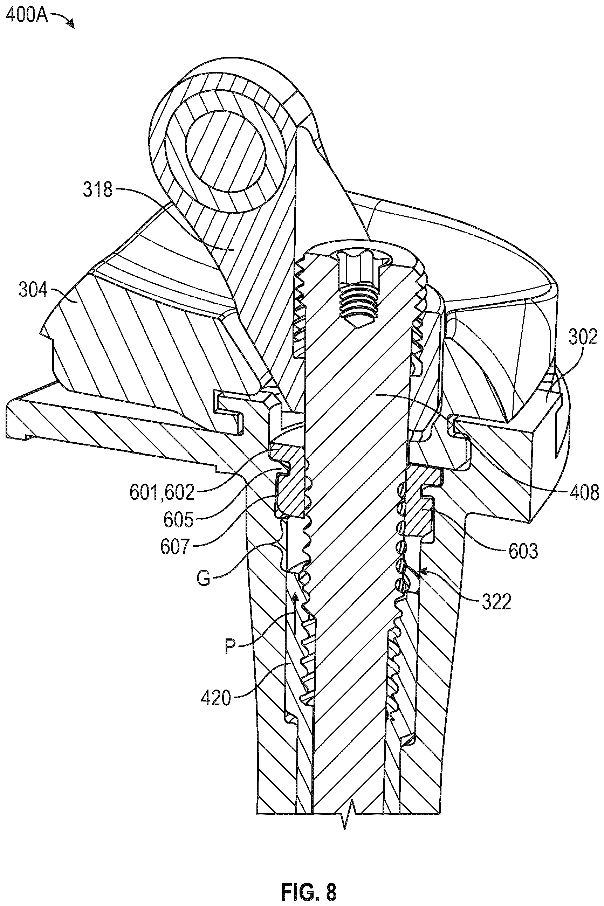

FIG. 7 shows various examples of capture elements 501A, 501B, 501C, 501D and 501E each configured as a snap-fit component 502. The capture elements 501A, 501B, 501C, 501D and 501E can be utilized in various examples of the present application in the manner of the capture element 401 described above to limit distraction. The capture elements 501A, 501B, 501C, 501D and 501E include a generally cylindrical main body portion 504 (only referenced in regard to capture element 501A). Some of the capture elements 501A, 501B, 501C, 501D and 501E such as the capture elements 501C and 501D can include a lateral opening 505 in addition to and communicating with the thru hole 421. In addition to the main body portion 504, most of the capture elements 501A, 501B, 501C, 501D and 501E can include legs 506 (only referenced in regard to capture element 501A) or other deflectable features configured to engage with mating features (grooves, projections, etc.) of the tibial baseplate (not shown). The legs 506 can include projections 508 and/or grooves or other features to facilitate snap-fit connection with the tibial baseplate as shown in FIGS. 5 and 8.

FIG. 8 shows a cross-section of a prosthesis assembly 400A including the tibial baseplate 302, part of the tibial bearing component 304, the hinge post 408 and the bushing 420. The hinge post 408 is not yet fully seated down into the bushing 420 and threadibly coupled with the shackle 318 as was the case with the example of FIG. 5. FIG. 8 shows a capture element 601 configured as a snap-fit component 602 similar to those previously described in FIG. 7. The capture element 601 is at least partially positioned within the recess 322 and has been deflected such that feet or prongs 603 and other features engage one or more retention features 604 of the tibial baseplate 302. These one or more retention features 604 include a projection 605 and grooves 607 that are positioned along an edge of the recess 322. The capture element 601 is configured to engage with and form a snap-fit connection with the tibial baseplate 302 thereby retaining the capture element 601 to the tibial baseplate 302.

It should be noted that with FIG. 8, as the hinge post 408 is threaded to the shackle 318 threading between the bushing 420 and the middle portion of hinge post 408 can engage. This threading can be configured in the manner of the threading previously described in regard to FIG. 5. Threaded engagement can reposition the bushing 420 within the recess 322 as desired. For example, the bushing 420 can move in a proximal direction as indicated with arrow P to create a desired gap G between the bushing 420 and the capture element 601. The size of the gap G can dictate the amount of distraction of the femoral component (not shown) from the tibial bearing component 304 and the tibial baseplate 302. Distraction can be limited (halted) by contact between the bushing 420 (or hinge post 408) with the capture element 601 upon movement of the bushing 420 with the hinge post proximally.

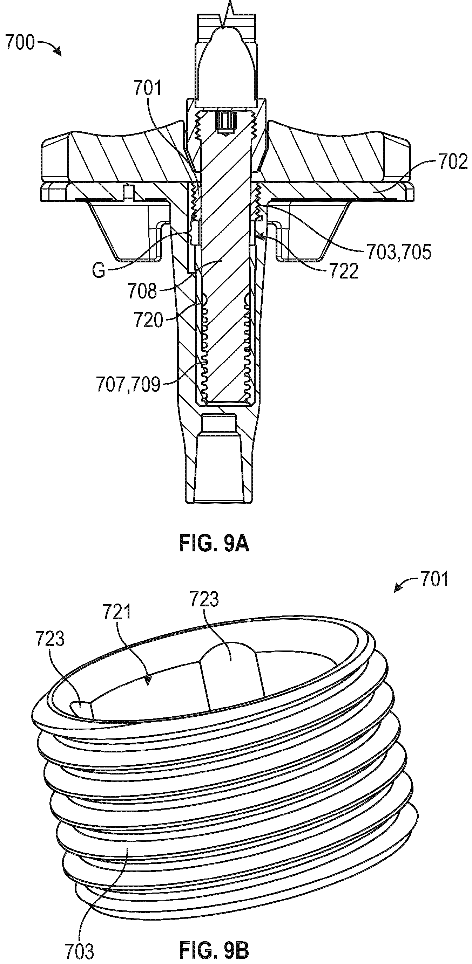

FIG. 9A shows a cross-section of another example of a capture element 701 being used with a prosthesis assembly 700. The capture element 701 can include threads 703 configured to couple with corresponding threads 705 along a recess 722 of a tibial baseplate 702. The capture element 701 is captured at least partially within the recess 722 once threaded into position as shown in FIG. 9A. FIG. 9A additionally shows further examples of a hinge post 708 and a bushing 720. The hinge post 708 can have a construction similar to that of the hinge post 408 but can have threads 707 along a distal portion 709 thereof rather than a middle portion as in the example of FIG. 5. The bushing 720 and the hinge post 708 can couple together by engaging threads in a manner similar to FIG. 5.

FIG. 9B shows the capture element 701 with the external threads 703 along an outer diameter along with a thru hole 721 similar to those described previously. The thru hole 721 can include drive features 723 such as grooves, lobes or the like for mating engagement by a driver (screw driver) or other tool used to thread the capture element 701 onto the tibial baseplate 702 to capture the bushing 720 and/or a distal feature of the hinge post 708.

Similar to FIG. 8, FIG. 9A shows the gap G between the bushing 720 and the capture element 701. The size of the gap G can dictate the amount of distraction of the femoral component (not shown) from the tibial bearing component 304 and the tibial baseplate 702. Distraction can be limited (halted) by contact between the bushing 720 (or hinge post 708) with the capture element 701 upon movement of the bushing 720 with the hinge post 708 proximally. The position of the bushing 720 within the recess 722 can be adjusted as previously described by the threaded engagement between the bushing 720 and the hinge post 708 (e.g., via small differences in pitch or other geometry of the threads, for example).

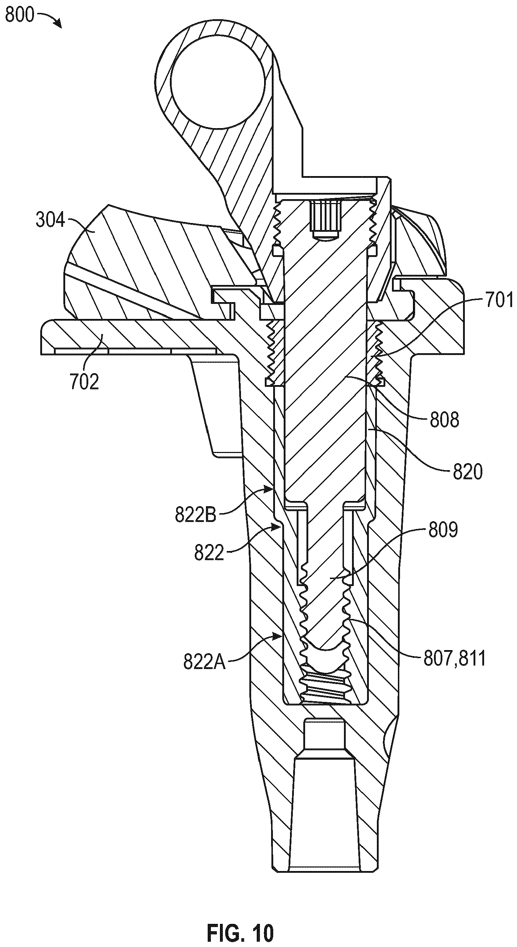

FIG. 10 shows a cross-section of a prosthesis assembly 800 including the tibial baseplate 702, part of the tibial bearing component 304 and the capture element 701 previously shown in FIG. 9A. However, the example of FIG. 10 includes a hinge post 808 and a bushing 820 having a modified shape from that of FIG. 9A. Thus, FIG. 10 additionally shows further examples of the hinge post 808 and the bushing 820. The hinge post 808 can have a construction similar to that of the hinge post 408 but can have threads 807 along a smaller diameter distal portion 809 thereof rather than a middle portion as in the example of FIGS. 5 and 8. The bushing 820 can have a counter-bore or configuration providing for a reduced volume at a distal portion as compared with a proximal portion. Thus, a distal recess 822A can have a smaller diameter as compared with a more proximal recess 822B of the recess 822. The hinge post 808 can couple together by engaging threads 811 of the bushing 820 in a manner similar to FIG. 5.

In FIG. 10, the bushing 820 can be configured to entirely limit distraction of the femoral component (not shown) from the tibial bearing component 304 and the tibial baseplate 702. Thus, substantially no distraction of the femoral component is possible. This is a result of the length of the bushing 820 being elongated relative to a length of the recess 822 such that that bushing 820 can abut with the capture element 701 as shown. Thus, there is no gap G (FIGS. 8 and 9A) between the bushing 820 and the capture element 701. Distraction is limited (halted) by contact between the bushing 820 (or hinge post 808) with the capture element 701.

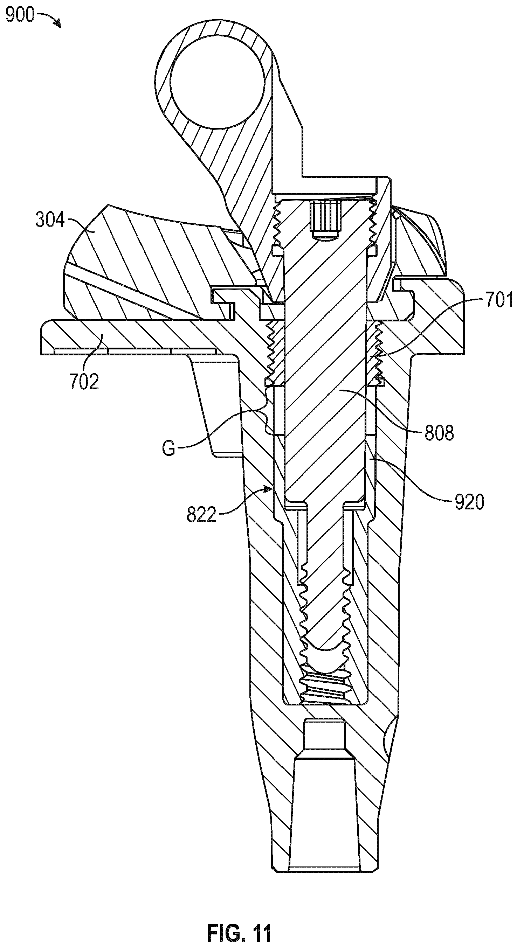

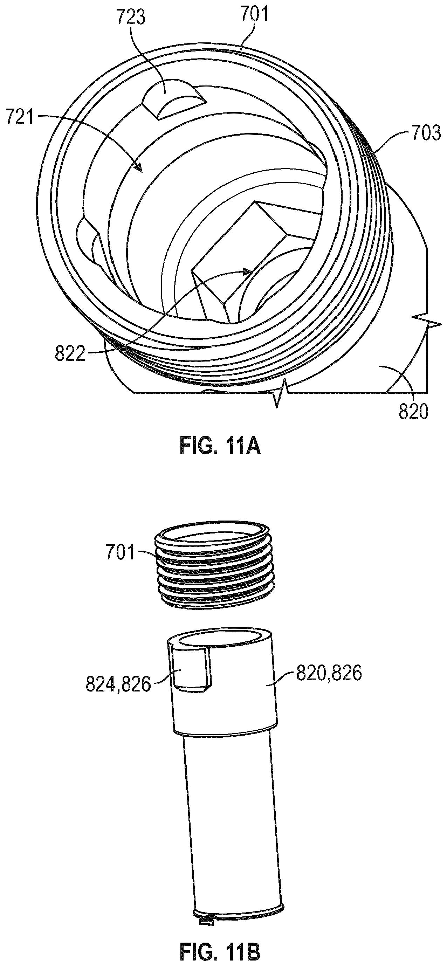

FIG. 11 shows a cross-section of a prosthesis assembly 900 including the tibial baseplate 702, part of the tibial bearing component 304, the hinge post 808 and the capture element 701 previously shown in FIGS. 9A and 10. However, the example of FIG. 11 includes a bushing 920 having a modified shape from that of FIG. 10. In particular, the longitudinal length of the bushing 920 has been shortened relative to a longitudinal length of the recess 822 so as to provide for the gap G between the bushing 920 and the capture element 701. The size of the gap G dictates the amount of distraction of the femoral component (not shown) from the tibial bearing component 304 and the tibial baseplate 702. Distraction can be limited (halted) by contact between the bushing 920 (or hinge post 708) with the capture element 701 upon movement of the bushing 920 with the hinge post 708 proximally. The position of the bushing 920 within the recess 822 can be adjusted as previously described by the threaded engagement between the bushing 820 and the hinge post 708 (e.g., via small differences in pitch or other geometry the threads, for example).

FIG. 11A is a perspective view of proximal portions of the capture element 701 and the bushing 920. The capture element 701 can have the external threads 703 along the outer diameter along with the thru hole 721. The thru hole 721 can include the drive features 723 as previously discussed. The recess 822 of the bushing 920 is shown with the counter-bore configuration described previously in FIG. 10.

FIG. 11B shows the relative spacing of the capture element 701 from the bushing 920 (prior to any limited distraction). The bushing 920 can include an anti-rotation feature 824 along an outer diameter 826 thereof. The anti-rotation feature 824 is shown as a lobe 828, for example. The anti-rotation feature 824 provides a stop to rotational movement of the bushing 920. However, other types of anti-rotation features such as thread, projections, tabs, prongs, barbs, ribs, etc. are contemplated. The anti-rotation feature 824 can be used with any of the bushing designs shown and discussed herein (including the bushing 320 of FIG. 4), and thus, is not limited to the bushing 920.

FIG. 12 shows a cross-section of a prosthesis assembly 1000 including the tibial baseplate 702, part of the tibial bearing component 304, the bushing 820 and the capture element 701 previously shown in FIGS. 9A and 10. However, the example of FIG. 12 includes a hinge post 1008 having a modified shape from that of FIG. 10. In particular, the hinge post 1008 can include one or more engagement features 1001 that can be configured to engage with the capture element 701 after a limited distraction by the femoral component (not shown). The one or more engagement features 1001 can be a tab, rib or other type of projection. The one or more engagement features 1001 can be passed through the thru hole 721 (FIG. 11A) such as by passing along one or more of the drive features 723 and then can be clocked (rotated) into a different position as shown in FIG. 12. The gap G can exist between the one or more engagement features 1001 and a distal end of the capture element 701. The size of the gap G can dictate the amount of distraction of the femoral component (not shown) from the tibial bearing component 304 and the tibial baseplate 702. Distraction can be limited (halted) by contact between the one or more engagement features 1001 (part of the hinge post 1008) with the capture element 701. Thus, the configuration of the hinge post 1008 itself can limit distraction rather than any contact between the bushing 820 and the capture element 701.

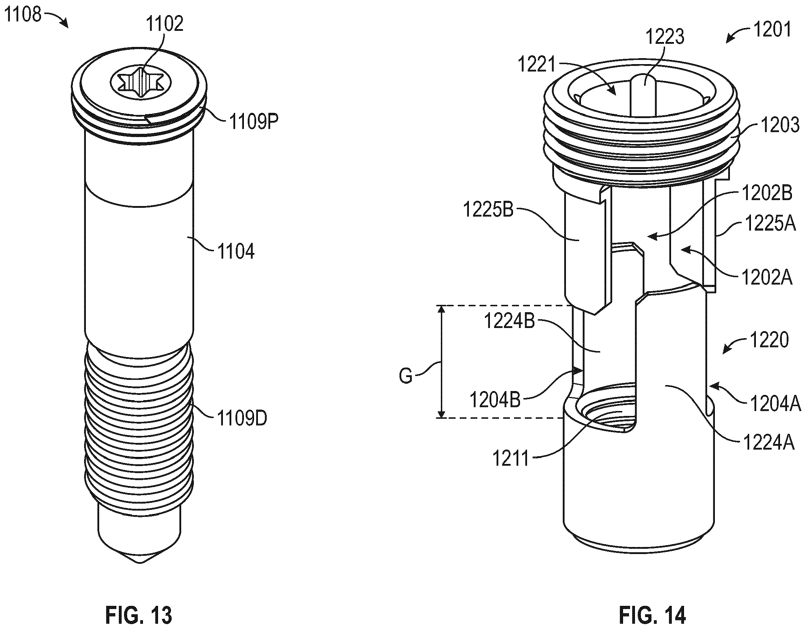

FIG. 13 is a perspective view of a hinge post 1108 according to another example. The hinge post 1108 can include posterior threads 1109P and distal threads 1109D. The hinge post 1108 can additionally include a driving feature 1102 at a proximal end and an unthreaded intermediate portion 1104. The threads 1109P and/or 1109D can be rope threads, knuckle threads, ACME threads or other types of threads as known in the art. The intermediate portion 1104 can be positioned between the posterior threads 1109P and the distal threads 1109D. The intermediate portion 1104 and/or other portions can be slightly tapered, for example.

FIG. 14 is a perspective view of a capture element 1201 and a bushing 1220 with a relative spacing prior to any limited distraction of the knee joint. The bushing 1220 includes threads 1211 configured to engage with the threads 1109D (FIG. 13) of the hinge post 1108, for example. The bushing 1220 additionally includes two anti-rotation features 1224A and 1224B. The capture element 1201 can include external threads 1203, a thru hole 1221, drive features 1223, and two anti-rotation features 1225A and 1225B. The capture element 1201 can have the external threads 1203 along the outer diameter along with the thru hole 1221. The thru hole 1221 can include the drive features 1223 as previously discussed.

The two anti-rotation features 1224A and 1224B can be configured to engage the corresponding two anti-rotation features 1225A and 1225B. This can occur during distraction and/or rotation of the femoral component, the hinge post 1108 (FIG. 13) and the bushing 1220. The anti-rotation features 1224A and 1224B can be proximally extending prongs or other features that are insertable into suitably shaped recesses 1202A and 1202B during limited distraction of the hinge post 1108 (FIG. 13) and the bushing 1220. This limited distraction can bring the bushing 1220 relatively closer to the capture element 1201 than the spacing shown in FIG. 14. The suitably shaped recesses 1202A and 1202B can be formed between the two anti-rotation features 1225A and 1225B. Similarly, the two anti-rotation features 1225A and 1225B are insertable into suitably shaped recesses 1204A and 1204B of the bushing 1220 during limited distraction of the hinge post 1108 (FIG. 13) and the bushing 1220. The recesses 1202A and 1202B can be shaped and sized relative to the anti-rotation features 1224A and 1224B and the recesses 1204A and 1204B can be shaped and sized relative to the anti-rotation features 1225A and 1225B to allow for a desired limited rotation (e.g., 30 degrees or less) of the hinge post 1108 (FIG. 13), the bushing 1220 and the femoral component (not shown). Such rotation can be halted by engagement between two or more of the anti-rotation features 1224A, 1224B, 1225A and/or 1225B.

The spacing between the capture element 1201 and the bushing 1220 can comprise the gap G discussed previously. The size of the gap G dictates the amount of proximal distraction of the femoral component (not shown) from the tibial bearing component and the tibial baseplate. Additionally, a rotation gap can be provided between the anti-rotation features 1224A, 1224B, 1225A and 1225B to limit rotation of the femoral component. Distraction can be limited (halted) by contact between the bushing 1220 with the capture element 1201 upon movement of the bushing 1220 with the hinge post 1108 (FIG. 13) proximally and/or in rotation.

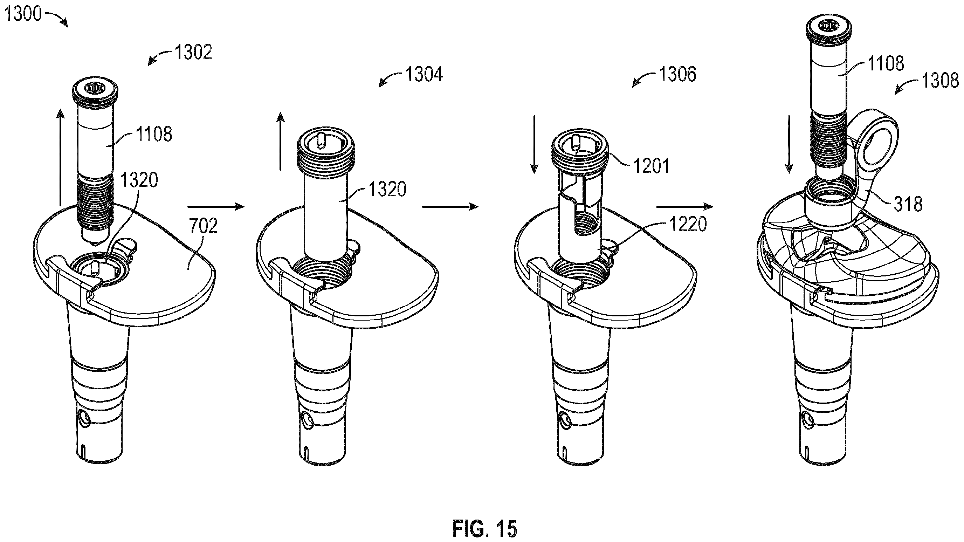

FIG. 15 illustrates a process of switching a constrained knee prosthesis from a bushing 1320 (shown to viewer's left) that allows for full distraction to the bushing 1220 and the capture element 1201 (shown to viewer's right) that provides from limited distraction according to an example of the present application. The method 1300 can include removing at step 1302 the hinge post 1108 from the bushing 1320 and from the tibial baseplate 702. The method 1300 removes, at step 1304, the bushing 1320 configured for the full distraction of the knee joint from the tibial baseplate 702. The bushing 1220 and the capture element 1201 are inserted at step 1306 into the tibial baseplate 702. The hinge post 1108 at step 1308 can then be coupled to the shackle 318 and reinserted back into the tibial baseplate 702 coupling with the bushing 1220 and the capture element 1201 to provide for limited distraction of the knee joint as discussed herein.

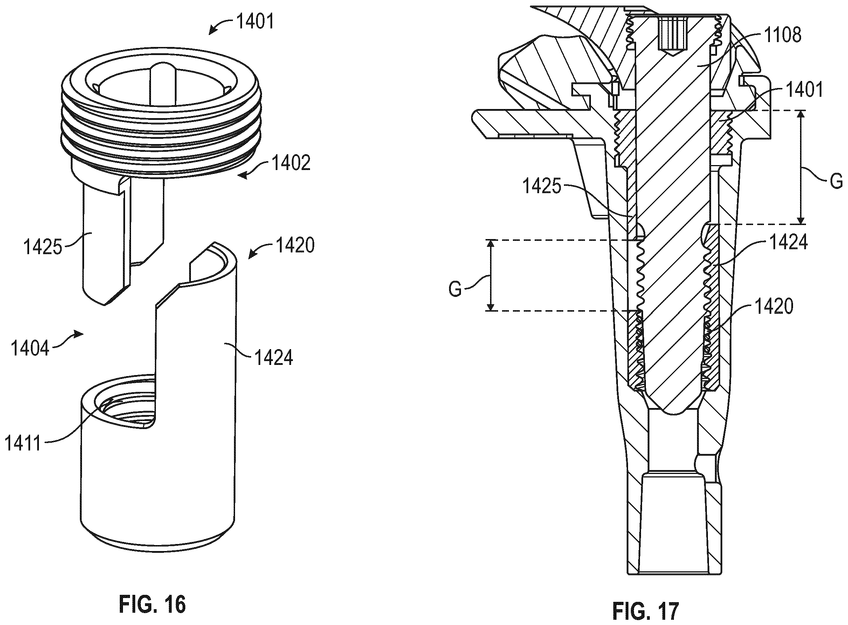

FIG. 16 is a perspective view of a capture element 1401 and a bushing 1420 with a relative spacing prior to any limited distraction of the knee joint. The bushing 1420 includes threads 1411 configured to engage with the threads 1109D (FIG. 13) of the hinge post 1108, for example. The bushing 1420 can be configured in the manner of the bushing 1220 of FIGS. 14 and 15 discussed previously. The bushing 1420 can differ in that a single anti-rotation feature 1424 is utilized. The capture element 1401 can include a single anti-rotation feature 1425.

The anti-rotation feature 1424 can be configured to engage the corresponding anti-rotation feature 1425. The anti-rotation feature 1424 can be a proximally extending prong or other feature that are insertable into suitably shaped recess 1402 of the capture element 1401 during limited distraction of the hinge post 1108 (FIG. 13) and the bushing 1420. This limited distraction can bring the bushing 1420 relatively closer to the capture element 1401 than the spacing shown in FIG. 16. The suitably shaped recess 1402 can be opposing the anti-rotation feature 1424 and can be partially formed by opposing edges of the anti-rotation feature 1425. Similarly, the anti-rotation feature 1425 can be insertable into a suitably shaped recess 1404 of the bushing 1420 during limited distraction of the hinge post 1108 (FIG. 13) and the bushing 1420. The recess 1402 can be shaped and sized relative to the anti-rotation feature 1424 and the recess 1404 can be shaped and sized relative to the anti-rotation feature 1425 to allow for a desired limited rotation (e.g., 30 degrees or less) of the hinge post 1108 (FIG. 13), the bushing 1220 and the femoral component (not shown). Engagement of the anti-rotation feature 1424 with the anti-rotation feature 1425 can halt the rotation, thus, providing for limited rotation of the knee joint.

FIG. 17 shows the spacing between the capture element 1401 and the bushing 1420 can comprise the gap G discussed previously. The size of the gap G dictates the amount of proximal distraction of the femoral component (not shown) from the tibial bearing component and the tibial baseplate. Additionally, a rotation gap (FIG. 16) can be provided between the anti-rotation features 1424 and 1425 to limit rotation of the femoral component. Distraction can be limited (halted) by contact between the bushing 1420 with the capture element 1401 upon movement of the bushing 1420 with the hinge post 1108 proximally and/or in rotation.

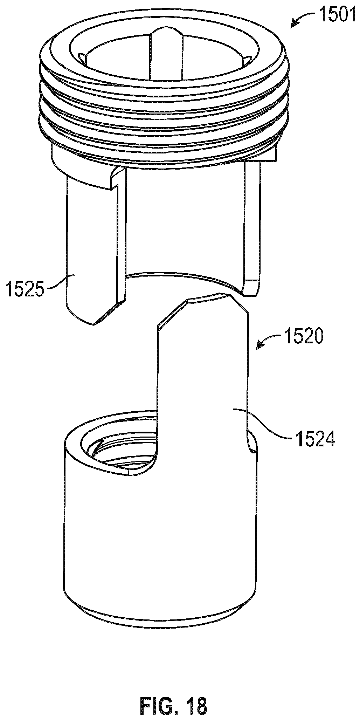

FIG. 18 shows a capture element 1501 and a bushing 1520 with a relative spacing prior to any limited distraction of the knee joint. The bushing 1520 and the capture element 1501 are constructed in a manner very similar to that of the examples of FIG. 16. The size of anti-rotation feature 1524 has been modified to be smaller than the example of FIG. 16 while anti-rotation feature 1525 has been enlarged. Anti-rotation features 1524 and 1525 can be suitably sized to control distraction and/or rotation of the knee joint as desired.

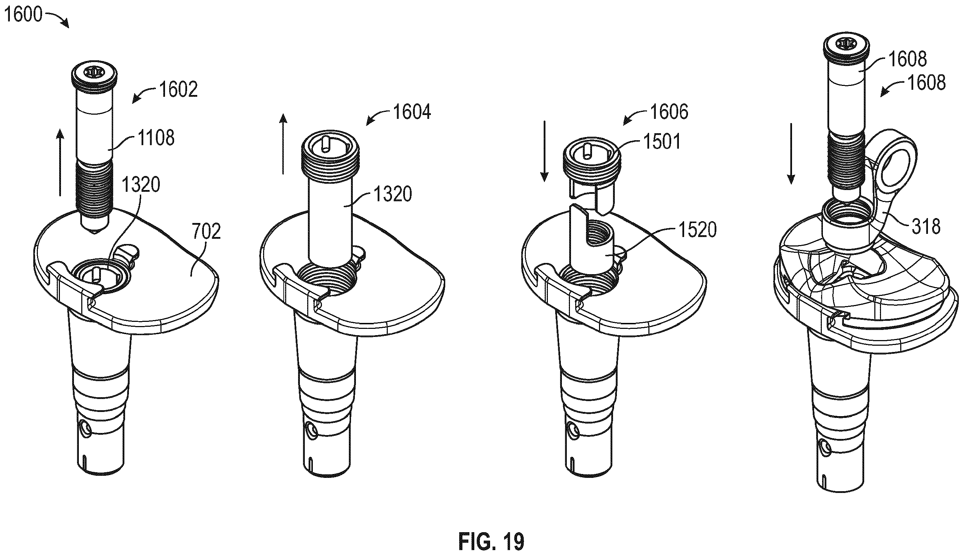

FIG. 19 illustrates a process of switching a constrained knee prosthesis from the bushing 1320 (shown to viewer's left) that allows for full distraction to the bushing 1520 and the capture element 1501 (shown to viewer's right) that provides from limited distraction according to an example of the present application. The method 1600 can include removing at step 1602 the hinge post 1108 from the bushing 1520 and from the tibial baseplate 702. The method 1600 removes, at step 1604, the bushing 1520 configured for the full distraction of the knee joint from the tibial baseplate 702. The bushing 1520 and the capture element 1501 are inserted at step 1606 into the tibial baseplate 702. The hinge post 1108 at step 1608 can then be coupled to the shackle 318 and reinserted back into the tibial baseplate 702 coupling with the bushing 1520 and the capture element 1501 to provide for limited distraction of the knee joint as discussed herein.

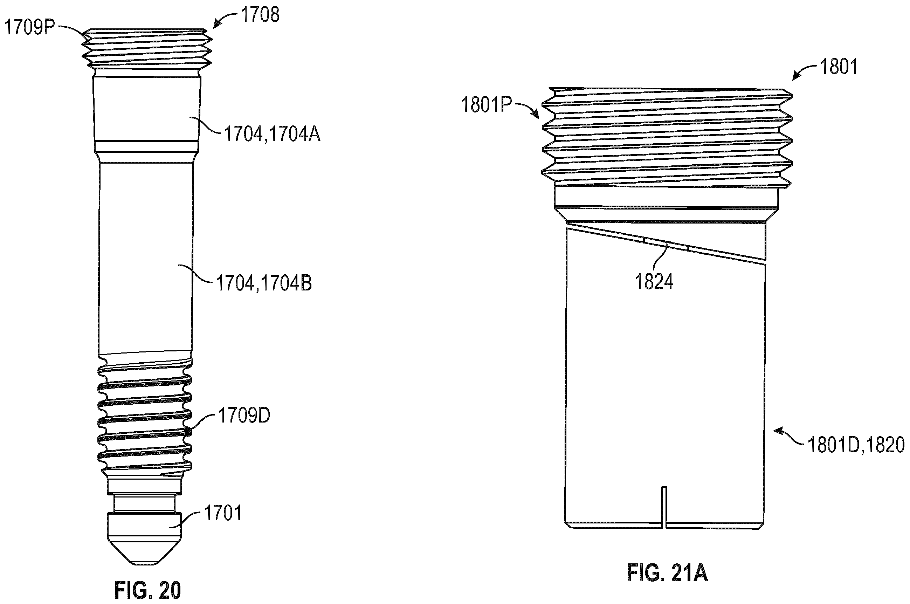

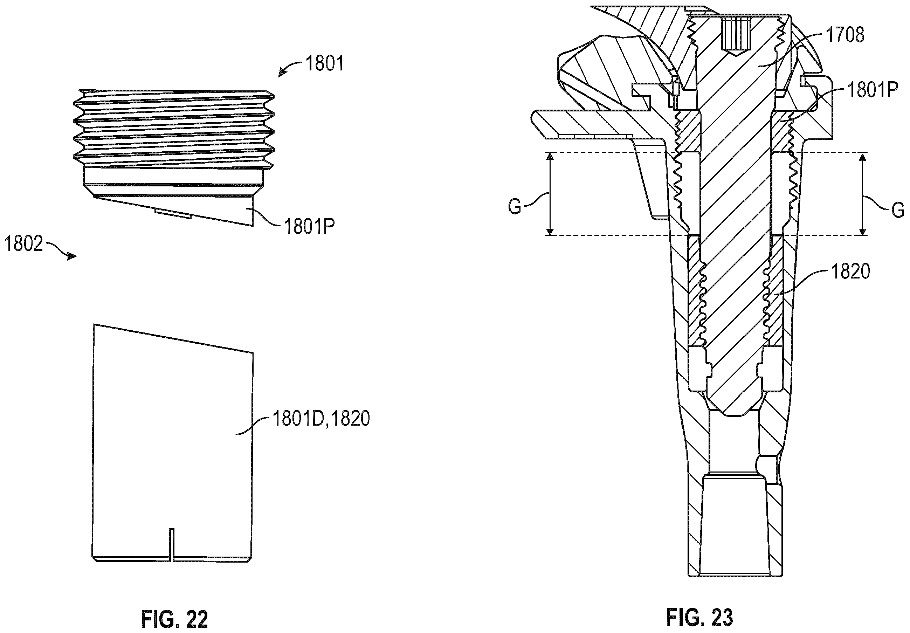

FIG. 20 is a perspective view of a hinge post 1708 according to another example. The hinge post 1708 can include posterior threads 1709P and distal threads 1709D. The hinge post 1708 can additionally include an unthreaded intermediate portion 1704 having a first diameter portion 1704A and a second smaller diameter portion 1704B. The threads 1709P and/or 1709D can be rope threads, knuckle threads, ACME threads or other types of threads as known in the art. The intermediate portion 1704 can be positioned between the posterior threads 1709P and the distal threads 1709D. The intermediate portion 1704 and/or other portions can be slightly tapered, for example. A distal end portion 1701 of the hinge post 1708 can be untreaded and can be configured to pass through and space a capture element (see subsequent FIGS.) from a distal end of a recess in the tibial baseplate as shown in FIG. 22.

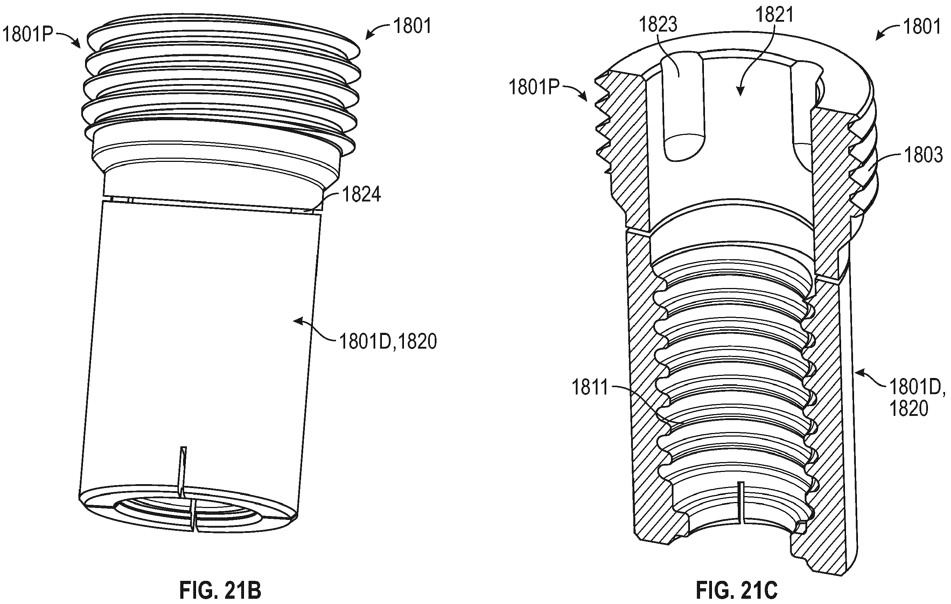

FIGS. 21A-21C show another example of a capture element 1801 that combines both a proximal capture element 1801P and a distal element 1801D that can act as a bushing 1820. The capture element 1801 is initially as a single component that include scoring, gap(s) or other features for defining (spacing) the proximal capture element 1801P from the distal element 1801D but also can have an intermediate connection element 1825 coupling the proximal capture element 1801P with the distal element 1801D. FIGS. 21A-21C show the capture element 1801 prior to threading engagement first with the hinge post (e.g., the hinge post 1708) and then with the tibial baseplate. In FIGS. 21A and 21B, the capture element 1801 is a single component with a frangible or separable connection provided by the intermediate connection element 1825 between the proximal capture element 1801P with the distal element 1801D.

As shown in the cross-sectional view of FIG. 21C, the distal element 1801D, the bushing 1820 has internal threads 1811 configured to engage with the threads 1709D (FIG. 20) of the hinge post 1708, for example. The proximal capture element 1801P has external threads 1803, a thru hole 1821 (shared with the distal capture element 1801D) and drive features 1823. The thru hole 1821 can include the drive features 1823 configured in a manner as previously discussed.

FIG. 22 shows the capture element 1801 with the proximal capture element 1801P separated from the distal element 1801D. This separation process occurs once the capture element 1801 is seated down partially into the recess of the tibial baseplate and then is engaged by threading of the hinge post 1708 into the distal element 1801D (e.g., the distal threads 1709D (FIG. 20) engage with the internal threads 1811 (FIG. 21C)). This threading acts to separate the proximal capture element 1801P from the distal element 1801D (severs/breaks/snaps the intermediate connection element 1825 of FIGS. 21A and 21B). The distal element 1801D threaded to the hinge post 1708 is carried further distally into the recess of the tibial baseplate and acts as the bushing 1820 similar to those discussed herein previously. The proximal capture element 1801P and the bushing 1820 can end up with the relative spacing shown in FIG. 22 allowing for some limited distraction of the knee joint.

This limited distraction can bring the bushing 1820 relatively closer to the proximal capture element 1801P than the spacing shown in FIG. 22. A recess 1802 can be shaped and sized to allow for a desired limited distraction of the hinge post 1708 (FIG. 20), the bushing 1820 and the femoral component (not shown).

FIG. 23 shows the spacing between the proximal capture element 1801P and the bushing 1820 can comprise the gap G discussed previously. The size of the gap G dictates the amount of proximal distraction of the femoral component (not shown) from the tibial bearing component and the tibial baseplate. Distraction can be limited (halted) by contact between the bushing 1820 with the proximal capture element 1801P upon movement of the bushing 1820 with the hinge post 1108 proximally.

### Additional Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "generally" "substantially" "about" mean within 15 percent of the value provided (±). The terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) can be used in combination with each other. Other examples can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above detailed description, various features can be grouped together to streamline the disclosure. This should not be interpreted as intending that an unclaimed disclosed feature is essential to any claim. Rather, inventive subject matter can lie in less than all features of a particular disclosed example. Thus, the following claims are hereby incorporated into the detailed description as examples or embodiments, with each claim standing on its own as a separate example, and it is contemplated that such examples can be combined with each other in various combinations or permutations. The scope of the invention should be determined with reference to the appended claims, along with the full scope of equivalents to which such claims are entitled.

## Claims

1. A prosthesis assembly for a constrained knee comprising:
a femoral component;
a tibial bearing component engaged by the femoral component;
a tibial baseplate having a distal surface, a proximal surface opposite the distal surface and facing the tibial bearing component, a periphery extending between the proximal surface and the distal surface and a keel extending distally from the distal surface;
a bushing received in a recess in the tibial baseplate;
a hinge post coupled to the femoral component and the bushing, wherein the hinge post is at least partially received by the bushing; and
a capture element coupled to the tibial baseplate, wherein the capture element is engaged by at least one of the bushing or the hinge post to limit distraction of the femoral component from the tibial bearing component and tibial baseplate.

2. The prosthesis assembly of claim 1, wherein the capture element comprises one of a snap fit component or a threaded nut configured to be at least partially received in the recess in the tibial baseplate, wherein the capture element has a thru hole configured to allow at least a portion of the hinge post to pass through the capture element.

3. The prosthesis assembly of any one of claims 1-2, wherein the capture element is spaced a distance proximal of the bushing, and wherein the distance allows for a limited degree of distraction of the femoral component from the tibial bearing component and tibial baseplate.

4. The prosthesis assembly of any one of claims 1-2, wherein the capture element is proximal to and abuts a proximal end of the bushing and thereby entirely limits distraction of the femoral component from the tibial bearing component and tibial baseplate.

5. The prosthesis assembly of any one of claims 1-4, wherein hinge post is coupled to the bushing by first threads that couple with second threads of the bushing.

6. The prosthesis assembly of claim 5, wherein coupling of the first threads with the second threads positions the bushing a desired location with respect to the capture element.

7. The prosthesis assembly of any one of claims 1-6, wherein the capture element is initially coupled to the bushing by a frangible connection, wherein the frangible connection is severed by threading of the hinge post with the bushing to separate the capture element from the bushing.

8. The prosthesis assembly of claim 1, wherein the capture element is configured to abut or is spaced a distance from the bushing when the bushing is positioned in the desired location.

9. The prosthesis assembly of claim 1, wherein, when assembled with the bushing, the capture element is proximal to and abuts a proximal end of the bushing and thereby entirely limits distraction of the femoral component from the tibial bearing component and tibial baseplate.

10. A method of assembling a prosthesis assembly for a constrained knee, the method comprising:
coupling a hinge post with a femoral component;
inserting a bushing into a recess in a tibial baseplate;
positioning a capture element within the recess of the tibial baseplate;
inserting the hinge post when coupled to the femoral component into the recess and through the capture element;
coupling the hinge post with the bushing within the recess; and
inserting a tibial bearing component between the femoral component and the tibial baseplate.

11. The method of claim 10, wherein positioning the capture element within the recess of the tibial baseplate includes one of: deflecting the capture element to engage one or more retention features of the tibial baseplate or threading the capture element into the tibial baseplate.

12. The method of claim 10, wherein positioning the capture element within the recess of the tibial baseplate includes inserting the capture element into a receptacle that is adjacent to and communicates with the recess and moving the capture element laterally from the receptacle to the recess.

13. The method of any one of claims 10-12, wherein coupling the hinge post with the bushing within the recess positions the bushing at a desired location relative to the capture element and further comprising engaging the capture element with at least one of the bushing or the hinge post to limit distraction of the femoral component from the tibial bearing component and tibial baseplate.

14. The method of any one of claims 10-13, wherein coupling the hinge post with the bushing within the recess severs a connection between the capture element and the bushing.
